# EUROPEAN PATENT SPECIFICATION

(11) **EP 1 202 727 B1**
(45) Date of publication and mention of the grant of the patent: **29.12.2004**
(21) Application number: 00947901.5
(22) Date of filing: 30.06.2000
(51) Int. Cl.: A61K 31/197, A61K 31/662, A61P 25/34

(54) **USE OF AGONISTS OF GABAB RECEPTORS AND PHARMACEUTICALLY ACCEPTABLE DERIVATIVES THEREOF, IN THE THERAPY OF MAINTAINING NICOTINE ABSTINENCE IN NICOTINE-DEPENDENT PATIENTS**
VERWENDUNG VON GABAB-REZEPTOR AGONISTEN UND DEREN PHARMAZEUTISCH AKZEPTIERBAREN DERIVATE BEI DER THERAPIE ZUR ERHALTUNG VON NIKOTINABSTINENZ BEI NIKOTINABHÄNGIGEN PATIENTEN
UTILISATION D'AGONISTES DE RECEPTEURS GABA B? ET DE LEURS DERIVES ACCEPTABLES SUR LE PLAN PHARMACEUTIQUE DANS LA THERAPIE DE MAINTIEN DE L'ABSTINENCE A LA NICOTINE CHEZ LES PATIENTS DEPENDANTS DE LA NICOTINE

(30) Priority: 30.07.1999 IT MI991715
(43) Date of publication of application: 08.05.2002
(73) Proprietor: L. MOLTENI & C. DEI FRATELLI ALITTI SOCIETA' DI ESERCIZIO S.P.A, 50018 Scandicci (IT)
(72) Inventor: GESSA, Gian, Luigi, I-09124 Cagliari (IT); COLOMBO, Giancarlo, I-09125 Cagliari (IT); PANI, Luca, I-09126 Cagliari (IT); FRATTA, Walter, I-09047 Selargius (IT)
(74) Representative: Gervasi, Gemma, Dr.
(86) International application number: PCT/EP2000/006120
(87) International publication number: WO 2001/008675

(56) References cited:
- EP-A- 0 439 430
- WO-A-00/07583
- US-A- 4 126 684
- US-A- 5 760 049
- DEWEY S L ET AL: "A PHARMACOLOGIC STRATEGY FOR THE TREATMENT OF NICOTINE ADDICTION" SYNAPSE,GB,WILEY AND SONS, CHICHESTER, vol. 31, no. 1, January 1999 (1999-01), pages 76-86, XP000856613 ISSN: 0887-4476
- BULLOCK AMY E ET AL: "Nicotine tolerance in chromaffin cell cultures: Acute and chronic exposure to smoking-related nicotine doses." JOURNAL OF NEUROCHEMISTRY, vol. 62, no. 5, 1994, pages 1863-1869, XP000965643 ISSN: 0022-3042
- OSET-GASQUE M J ET AL: "GABA-B receptors modulate catecholamine secretion in chromaffin cells by a mechanism involving cyclic AMP formation." BRITISH JOURNAL OF PHARMACOLOGY, vol. 110, no. 4, 1993, pages 1586-1592, XP000965271 ISSN: 0007-1188
- CASTRO E ET AL: "GABA-A AND GABA-B RECEPTORS ARE FUNCTIONALLY ACTIVE IN THE REGULATION OF CATECHOLAMINE SECRETION BY BOVINE CHROMAFFIN CELLS" JOURNAL OF NEUROSCIENCE RESEARCH, vol. 23, no. 3, 1989, pages 290-296, XP000965416 ISSN: 0360-4012
- KERR DAVID I B ET AL: "3-Amino-2-(4-chlorophenyl)nitropropane is a new GABA-B receptor agonist, more active peripherally." EUROPEAN JOURNAL OF PHARMACOLOGY, vol. 236, no. 2, 1993, pages 239-245, XP000965318 ISSN: 0014-2999
- ROBERTS DAVID C S ET AL: "Baclofen suppression of cocaine self-administration: Demonstration using a discrete trials procedure." PSYCHOPHARMACOLOGY, vol. 131, no. 3, 1997, pages 271-277, XP000965272 ISSN: 0033-3158
- BROADBENT JULIE ET AL: "Differential effects of GABAA and GABAB agonists on sensitization to the locomotor stimulant effects of ethanol in DBA/2 J mice." PSYCHOPHARMACOLOGY, vol. 141, no. 2, January 1999 (1999-01), pages 197-205, XP000965275 ISSN: 0033-3158
- DATABASE CHEMABS [Online] CHEMICAL ABSTRACTS SERVICE, COLUMBUS, OHIO, US; CORRIGALL, WILLIAM A. ET AL: "Response of nicotine self-administration in the rat to manipulations of mu-opioid and.gamma.-aminobutyric acid receptors in the ventral tegmenta area" retrieved from STN Database accession no. 133:68829 XP000965273 & PSYCHOPHARMACOLOGY (BERLIN) (2000), 149(2), 107-114 ,
- FATTORE L ET AL: "Effect of baclofen on intravenous self-administration in mice and rats." EUROPEAN JOURNAL OF NEUROSCIENCE, vol. 12, no. Supplement 11, 2000, page 204 XP000965219 Meeting of the Federation of European Neuroscience Societies;Brighton, UK; June 24-28, 2000 ISSN: 0953-816X

## Description

### Field of the invention

The use of agonists of GABA_{B} receptors and pharmaceutically acceptable derivatives thereof, in reducing the wish of smoking in nicotine-dependent patients in treatment for maintaining nicotine abstinence is described.

### Background of the invention

The habit of smoking generally starts in youth. It is not clear how fast dependence develops, anyhow among those who successfully stop smoking less then 25% succeed at the first attempt.

The damages of tobacco smoke are well known. Nicotine, a tobacco alkaloid, is probably the only compound endowed of rewarding properties among the 4000 components of cigarette smoke, which, on the contrary, burns toxic substances. which are non-satisfying but pharmacologically very active.

Tobacco smoke contains: cigarette constituents (organic products, nicotine alkaloids, additives) and products deriving from combustion (CO₂, CO, tar). Some of the compounds contained in tar, and in particular products of combustion, are the actual responsible of various forms of cancer, especially pulmonary cancer. Among the active compounds of smoke, carbon monoxide and nicotine play an important role in determining cardiovascular diseases. Nicotine, in fact, has a direct action on cholinergic receptors and starts a cascade of pharmacological actions, linked to the liberation of catecholamines (adrenaline, noradrenaline, and dopamine) which can cause serious cardiovascular problems (atherosclerosis and pathologies of the heart).

In man the action of nicotine alters the nervous system physiology, both centrally and peripherally. Nicotine is capable of modifying the transmission of nervous impulses through a specific action on the sympathetic nervous system: at low concentration the nervous transmission is stimulated while at higher concentrations it is blocked.

Tolerance to nicotine is shown by the absence, in smokers, of nausea, vertigo and other classical symptoms which appear after administration of nicotine to non-smokers or by a lowering of the effect observed after the prolonged use of the same quantity of products containing nicotine.

The difficulty for smokers to stop smoking is due, partially, to the satisfying effects typical of nicotine, which is in fact under all aspects an abused substance (drug) and as such acts as a positive primary reinforce, i.e. causes rewarding sensations in the user, compelling him/her to repeated self-administration in order to attain continuously satisfying effects. Nicotine deprivation in strong smokers causes irritability and discomfort which can be overcome only by administering nicotine. Various symptoms of abstinence (irritability, anxiety, hunger, stimulus to smoke) are nicotine-specific. Among the various symptoms described in the literature the following should be mentioned, as they are important in determining a relapse: mood variation, irritability, anxiety, depression, somnolence, headache, breath difficulties, pains, limbs tingling, stomach-ache. This syndrome is often accompanied by signs of a bradycardia, peripheral flushing, decrease of urinary adrenaline, noradrenaline and cortisol, EEG alterations, variation of the endocrine functions, cognitive deficits such as disturbances in memory, attention and vigilance, sleep disorder, constipation, sweating, oral ulcers and cough. Medicament at present available for the treatment of smoke dependence can be subdivided in:

### Nicotine substituting systems

There are at list four different kind which have been tested: nicotine chewing gums, transdermal nicotine, nasal spray nicotine and nicotine for inhalation. All these systems attempt to obtain a progressive reduction of nicotine consumption with respect to the beginning of the treatment.. For three of the above said substituting systems [i.e. steam with nicotine (base for inhalation), nicotine nasal gel (now available also in spray) and nicotine chewing gum (2 mg)] the time requested to reach a clinically significant level is in the order of minutes, while in the case of transdermal nicotine the plateau level is attained after 8 hours.

### Symptomatic treatments

The efficacy of pharmacological treatments for symptoms and signs of abstinence and/or craving is demonstrated in the case of other substances of abuse. Fluoxetine for alcohol and clonidine for heroin. The same approach is applied to the treatments for stopping smoking.

The only nicotine antagonist proposed for stopping the smoking habit is mecamilamine, usually employed for the initial control of blood pressure in dissecting aorta aneurysm, however, it presents several central and peripheral side effect and has not demonstrated a convincing long-term clinical efficacy in maintaining nicotine abstinence.

### Deterrents and chemical surrogate of smoking

Numerous attempts performed with compounds which, added to cigarettes, would change their taste (silver salts) or which are inactive nicotine agonists (Lobeline) or have, themselves, rewarding properties different from nicotine, represent all potential alternatives able to induce conditioned reinforcement, did not however give satisfactory results.

### Behavioural or non pharmacological treatments

Many different therapies of this type are available; they include individual expedients, program for the behavioural modification, such as procedures which induce conditioned disgust, specialised psychological support and alternative therapies such as hypnosis and acupuncture.

In spite of an apparent high rate of smoking cessation at the beginning of treatment, the behavioural therapies do not appear to be very successful in the long run.

The difficulty of finding valid pharmacological therapies against the abuse of substances like nicotine, cocaine, heroin, alcohol etc. is due in part to their strong reinforcing effects. Such effects, which from the behavioural point of view are called positive hedonic effects, induce a "sensation", a "desire", which pushes the toxic patients or the experimental animal with unrestricted access to the drug (in the case of legal substance such as nicotine), to behave as driven by a compulsive wish of obtaining the substance which represents a fundamental component in the failure to Quit from smoking.

In WO 0007583 published on 17.02.2000 and claiming the priorities 05.08.1998, 09.11.1998 and 11.12.1998, it is described a method for changing addition-related behaviour of a mammal suffering from addiction to abused drugs by administering to the mammal i.a. γ-vinyl GABA (GVG) or γ-hydroxybutyric acid (GHB).

### Summary

The present invention refers to the use of agonists of GABA_{B} receptors as defined in the claims pharmaceutically acceptable derivatives thereof, for the preparation of pharmaceutical compositions for the treatment of nicotine or smoking abstinence and dependence in nicotine-dependant patients.

### Description of the invention

The Applicant has now found that agonists of GABA_{B} as defined in the claims, or pharmaceutically acceptable derivatives thereof, are capable of inhibiting the "craving" for nicotine in smoking patients.

With agonists of GABA_{B} according to the present invention it is intended the compounds, known in literature, capable of behaving in vivo as GABA_{B} agonists, for example: β-(4-chlorophenyl)GABA (hereinafter indicated with its trivial name: Baclofen), 3-aminopropyl(methyl)phosphinic acid (SKF 97541), 3-aminopropyl phosphinic acid (CGP 27492), γ-amino-β-4-(4-chlorophenyl)nitropropane
As pharmaceutically acceptable derivatives of the above said compounds it is intended, according to the invention, their salts, esters, ethers, complexes and their corresponding isomers.

Particularly preferred among the above said compounds is Baclofen which is a well known medicament having myorelaxant action, used in the treatment of spastic states.

More particularly the isomer R (-) Baclofen free base or equivalent compounds are preferred.

The above said compounds acting as agonist of GABA_{B} are normally worked in the form of capsules, pills, transdermal patches, sublingual gel, depot intramuscular preparations according to well known techniques.

For the preparation of the above reported formulations the usual appropriate additives or excipients are used; for example in the case of transdermal patches the more commonly used additives are: silica, mineral oil, polyethylene, polyester, ethylene, vinylacetate and other equivalent.

The average doses of a formulation for oral administration are comprised between five and fifty milligrams a day depending on the pharmaceutical preparation used. Specific examples of suitable formulation according to the invention is represented by:
capsules and sustained release pills for oral administration obtained by reserved systems or matrix systems, whose excipients are methylacrylate, polyethylene, polyvinylchloride, methylcellulose, carboxy-methyl-cellulose, lipides.
transdermal drug delivery (TDD) and transdermal therapeutic systems (TTS) such as:
   adhesive devices;
   reservoir devices;
   monolithic devices;

The excipients used in the above said preparations are normally: silicone, polyethylene, ethylenevinylacetate, mineral oil, adhesive polymers

It was surprisingly found that heavy smokers presenting muscular contractions of various origin (amyotrophyc lateral sclerosis, vertebral disc hernia, spastic wry-neck etc.) manifested a significant reduction of their smoking habit with an actual and dramatic spontaneous lowering in the number of nicotine cigarette consumed. A possible therapeutical protocol consists in the administration to the patient of 30-75 mg of active product per day, if preferred subdivided in three refracted times. The results of the tests reported hereinafter, where the effects of the administration of Baclofen were studied, show that Baclofen is an useful medicament capable of limiting the compulsive wish, and the "craving", which is at the basis of the nicotine dependence and addiction and which represents the principal cause of relapses.

### Clinic experimentation

The study was performed on 15 heavy smokers, having an age comprised between 30 - 65 years, showing nicotine dependence according to the DSM-IV criteria (1994).

All the patients have been treated for 6-8 weeks in mono-therapy with Baclofen 40 mg per day (range 30-75 mg). Five patients (about 30%) of the patients showed one or more of the following side effects due to the assumption of Baclofen (nausea, nightmares, headaches, sedation, vertigo) effects which immediately disappeared after reduction of the treatment. Only in one case it was necessary to suspend the administration of the medicament.

Almost all the patients (70%) showed a reduction of the subjective wish of smoking. Such effect was accompanied by a diminution of cigarettes smoked. In no case during the reduction of the voluntary assumption of tobacco the signs and symptoms typical of nicotine abstinence were noticed.

### Pharmacological experimentation

The detailed method has been elsewhere described by Martellotta et al. (1995)

### Animals

Albino male mice CD1 (Charles River, Italy) weighing 25-28 g were used in the test. The mice were housed 10 by cage and kept in a room with inverted night/day cycle (12 h light/dark), at 22°C with food and water *ad libitum*. All the experiments were performed between 9.00 and 13.00 a.m.

### Substances

The (-) nicotine, bitartrate salt (Sigma, Italy) was dissolved, immediately before the tests, in a physiological solution added with 1% heparin; the doses are referred to the salt. The (±) Baclofen (Tocris, UK) is dissolved in physiological solution and administered intraperitoneally (i.p.) in a volume of 1 ml/kg 20 minutes before the first session of self-administration. In tests on the blocking effect the CGP 35348 (0,25 and 50 mg/kg i.p.), an antagonist of GABA_{B} receptors, was administered 15 min. before Baclofen.

### Apparatus

A couple of mice are put, separately, in identical cages of Plexiglas (8x8x8 cm), each of which presents a frontal hole provided with a photocell activating a recorder (Coulbourn Instruments, USA) and automatic syringes. On the opposite side of the cage there is a fissure which permits to pass and fix the tail , a butterfly needle can be easily inserted in one of the lateral vein of the tail and the mouse venous system is connected with the syringes of the pump.

The significance was determined with one way variance analysis (ANOVA) followed by the test of Newman-Keuls.

Baclofen is capable of antagonising the effects of (-) nicotine in doses comprised between 0,625 and 2,5 mg/kg i.p.. Such effect was always suppressed by both doses of CGP 35348, demonstrating that the observed effect was mediated by receptors specific for GABA_{B}-type receptors.

From all the above said, it is evident that Baclofen, in an absolutely unexpected manner, showed how an agonist action on GABA_{B} receptors is capable of selectively suppressing the impulse to smoke in nicotine-dependent patients. Baclofen, and therefore the receptor system of GABA_{B} could control the behaviours which express the relapse in abuse, not only for those neurotransmitters which mediate the rewarding effect of smoking but also for those which are at the basis of the compulsive, and therefore irresistible, wish to restart smoking.

In view of the specific and selective effect on GABA_{B}-type receptors demonstrated in the antagonistic experiments with the compound CGP 35348, the invention must be intended to include also the use of pharmacologically equivalents of Baclofen or Baclofen isomers (salts, esters, complexes. etc.) or to other agonists of GABA_{B} receptor.

### Bibliography

DiChiara, G.; Imperato, A: Drugs abused by humans preferentially increased synaptic dopamine concentration in the mesolimbic system of freely moving rats. Proc. Natl. Acad. Sci. USA 85: 5274-5278; 1988.

Martellotta, M.C.; Kuzmin, A.; Zvartu, E. Cossu, G.; Gessa, G.L.; Fratta, W.: Isradipine inhibits nicotine intravenous self-administration in drug-naive mice. Pharmacol. Biochem. Behav. 52 (2): 271-274; 1995.

Imperato,A; Mulas, A.; DiChiara, G.: Nicotine preferentially stimulates dopamine release in the limbic system of freely moving rats. Eur.J.Pharmacol. 132:337-338; 1986.

Rowel, P.P.; Carr, L.A.; Gardner,.A.C.: Stimulation of [³H]dopamine release by nicotine in rat nucleus accumbens. J. Neurochem. 49: 1449-1454; 1987.

## Claims

1. Use of agonists of GABA_{B} receptors, or pharmaceutically acceptable derivatives thereof, for the preparation of pharmaceutical compositions for reducing the wish of smoking in nicotine-dependent patients in the treatment for maintaining nicotine abstinence **wherein such agonist of GABA**_{**B**} **is not the γ-hydroxy-butyric acid (GHB).**

2. Use according to claim 1, wherein the GABA_{B} receptors agonists are: β-(4-chlorophenyl)GABA (Baclofen), 3-aminopropyl(methyl)phosphinic acid, 3-aminopropylphosphinic acid, γ-amino-β-4-(4-chlorophenyl)nitropropane or pharmaceutically acceptable derivatives thereof selected from their salts, esters, ethers, complexes and their corresponding isomers.

3. Use according to Claim 2 wherein the GABA_{B} receptors agonist is Baclofen

4. Use according to claim 3, wherein the GABA_{B} receptors agonist is the isomer R(-) Baclofen free base.

5. Use according to Claim 1 wherein the pharmaceutical composition is in the form suitable for oral, parenteral, transdermal, depot administration.

6. Use according to Claim 5 wherein the suitable forms are: capsules, pills, transdermal patches, sublingual gel, depot intramuscular preparations.

7. Use according to Claim 6 wherein the pills are sustained release pills obtained by reserved systems or matrix systems.

8. Use according to Claim 5 wherein the transdermal composition are transdermal drug delivery and transdermal therapeutic systems.

9. Use according to Claim 8 wherein the formulations are: adhesive devices, reservoir devices, monolithic devices.

## Patentansprüche

1. Verwendung von Agonisten von GABA_{B}-Rezeptoren oder ihren pharmazeutisch brauchbaren Derivaten für die Herstellung pharmazeutischer Zusammensetzungen zur Verminderung des Wunsches zu Rauchen bei nicotinabhängigen Patienten bei der Behandlung zur Aufrechterhaltung von Nicotin-Abstinenz, wobei ein solcher GABA_{B}-Antagonist nicht die γ-Hydroxybuttersäure (GHB) ist.

2. Verwendung nach Anspruch 1, bei der die GABA_{B}-Rezeptoren-Agonisten β-(4-Chlorphenyl)GABA (Baclofen), 3-Aminopropyl(methyl)phosphinsäure, 3-Aminopropylphosphinsäure, γ-Amino-β-4-(4-chlorphenyl)nitropropan oder ihre pharmazeutisch brauchbaren Derivate sind, die aus ihren Salzen, Estern, Ethern, Komplexen und ihren korrespondierenden Isomeren ausgewählt sind.

3. Verwendung nach Anspruch 2, bei der der GABA_{B}-Rezeptoren-Agonist Baclofen ist.

4. Verwendung nach Anspruch 3, bei der der GABA_{B}-Rezeptoren-Agonist die freie Base des Isomers R-(-)-Baclofen ist.

5. Verwendung nach Anspruch 1, bei der die pharmazeutische Zusammensetzung in einer Form vorliegt, die für eine orale, parenterale, transdermale, Depot-Verabreichung geeignet ist.

6. Verwendung nach Anspruch 5, bei der die geeigneten Formen Kapseln, Tabletten, transdermale Pflaster, sublinguales Gel, intramuskuläre Depot-Zubereitungen sind.

7. Verwendung nach Anspruch 6, bei der die Tabletten Tabletten mit länger anhaltender Freisetzung sind, die durch Reservesysteme oder Matrixsysteme erhalten wird.

8. Verwendung nach Anspruch 5, bei der die transdermalen Zusammensetzungen transdermale Wirkstoffabgabe-Systeme und transdermale therapeutische Systeme sind.

9. Verwendung nach Anspruch 8, bei der die Formulierungen haftende Vorrichtungen, Reservoirvorrichungen, monolithische Vorrichtungen sind.

## Revendications

1. Utilisation d'agonistes des récepteurs GABA_{B}, ou leurs dérivés pharmaceutiquement acceptables, pour la préparation de compositions pharmaceutiques pour réduire l'envie de fumer de patients dépendant de la nicotine dans le traitement pour maintenir l'abstinence à la nicotine, **où ledit agoniste de GABA**_{**B**} **n'est pas l'acide γ-hydroxy-butyrique (GHB).**

2. Utilisation selon la revendication 1, où les agonistes des récepteurs GABA_{B} sont β-(4-chlorophényl)GABA (Baclofen), acide 3-aminopropyl(méthyl)phosphinique, acide 3-aminopropylphosphinique, γ-amino-β-4-(4-chlorophényl)nitropropane ou leurs dérivés pharmaceutiquement acceptables sélectionnés parmi leurs sels, esters, éthers, complexes et leurs isomères correspondants.

3. Utilisation selon la revendication 2, où l'agoniste des récepteurs GABA_{B} est Baclofen.

4. Utilisation selon la revendication 3, où l'agoniste des récepteurs GABA_{B} est la base libre de l'isomère R(-) Baclofen.

5. Utilisation selon la revendication 1, où la composition pharmaceutique est sous une forme appropriée pour l'administration orale, parentérale, transcutanée ou en dépôt.

6. Utilisation selon la revendication 5, où les formes appropriées sont: capsules, pilules, patchs transcutanés, gel sublingual, préparations intramusculaires en dépôt.

7. Utilisation selon la revendication 6, où les pilules sont des pilules à libération entretenue obtenues par des systèmes à réservoir ou des systèmes à matrice.

8. Utilisation selon la revendication 5, où les compositions transcutanées sont des systèmes de délivrance transcutanée du médicament et des systèmes thérapeutiques transcutanés.

9. Utilisation selon la revendication 8, où les formulations sont: des dispositifs adhésifs, des dispositifs formant réservoir et des dispositifs monolithiques.
